Europäisches Patentamt

European Patent Office

Office européen des brevets

(11) Publication number: 0 339 634
A1

# EUROPEAN PATENT APPLICATION

(21) Application number: 89107631.7

(51) Int. Cl.⁴: **A61K 7/15**

(22) Date of filing: 27.04.89

(30) Priority: 29.04.88 US 188208

(43) Date of publication of application:
02.11.89 Bulletin 89/44

(84) Designated Contracting States:
BE CH DE FR GB IT LI

(71) Applicant: ESTEE LAUDER INC.
767 Fifth Avenue
New York New York 10153(US)

(72) Inventor: Smith, Walter P.
89 Blackberry Drive
Stamford Connecticut 06903(US)
Inventor: Kavaliunas, Dalia R.
4 Midland Gardens
Bronxville New York 10708(US)
Inventor: Bevacqua, Andrew J.
23 Caleb Brewster Road
East Setauket New York 11733(US)

(74) Representative: Vossius & Partner
Siebertstrasse 4 P.O. Box 86 07 67
D-8000 München 86(DE)

(54) Post-foaming shave gel composition.

(57) Disclosed are (a) a post-foaming shave gel composition comprising water, a soap, a gelling agent, a post-foaming agent and a hyaluronic acid containing component selected from the group consisting of hyaluronic acid, hyaluronic acid containing polymers, salts of hyaluronic acid, salts of hyaluronic acid containing polymers and mixtures thereof, and (b) a method of shaving using such a composition. The composition of the invention is characterized by a combination of desirable properties, which to applicants' knowledge have not been achieved in prior art shave compositions.

EP 0 339 634 A1

## POST-FOAMING SHAVE GEL COMPOSITION

This invention relates to a post-foaming shave gel composition which includes a unique combination of ingredients, including a component from the group consisting of hyaluronic acid, hyaluronic acid containing polymers, salts of hyaluronic acid, salts of hyaluronic acid containing polymers, and mixtures thereof and a method of shaving using that composition. The composition of the invention provides a closer, less irritating shave than products currently available, and functions to counteract the redness and irritation commonly associated with shaving.

Aerosol shaving compositions have been available for more than thirty years. Such products characteristically form a lather as the composition is released from the aerosol container in which it is packaged. A major shortcoming of aerosol lathers is insufficient wetting of the skin and beard, which can result in an uncomfortable shave.

Some of the shortcomings of such aerosol shave products were said to be overcome by the post-foaming shave gels described in U.S. Patent 3,541,581. The post-foaming shave gels described in this patent are said to be dispensed from their containers as a stable gel substantially free from foaming. The gel is said to produce a self-generating foam after it is spread over the user's skin. The gel is also said to provide a lather with improved inter-bubble transport for improved wetting of the skin.

The shave gel described in the patent is not, however, entirely satisfactory. For one thing, it does not reduce skin irritation to as great an extent as desirable. There is, therefore, a need in the art for improved post-foaming shave gels that reduce skin irritation to a greater extent than do the post-foaming shave gels described in the patent. It would also be highly desirable if such improved post-foaming shave gels had additional desirable characteristics not found in the known composition, e.g., the ability to soften the skin.

An object of this invention is to provide an improved post-foaming shave gel which reduces skin irritation and redness caused by shaving to a greater extent than do the known compositions. This object is achieved by the composition of the invention comprising water, soap, a gelling agent, a post-foaming agent, and hyaluronic acid, a hyaluronic acid containing polymer, a salt of hyaluronic acid, a salt of a hyaluronic acid containing polymer or a mixture thereof as a hyaluronic acid containing component.

The compositions of the invention act to moisturize and to soften the beard and skin for a closer, smoother shave. In addition, the compositions of the invention tend not to be rinsed off the face, thereby continuing to moisturize and soften the skin after shaving.

The shave gel of the invention preferably also contains allantoin, aloe vera gel, a botanical plant extract or a mixture thereof as moisturizing and soothing agents. The inclusion of one or more of these agents provides for even less inflammatory shaves.

The present invention also comprises a method of shaving using the composition of the invention. In that method of shaving, an effective amount of the composition is applied to the user's beard under conditions under which the shave gel foams and thereafter the user's beard is shaved with, e.g., a straight razor or a safety razor.

The composition of the invention preferably comprises about 40-90% by weight water, about 4-35% of soap, about 0.01-5% by weight of gelling agent, about 0.5-12% by weight of a post-foaming agent, about 0.01-5% by weight of the hyaluronic acid containing component, and about 0.01-5% by weight of one or more of the moisturizing and soothing agents identified above.

Hyaluronic acid is a known naturally occurring polymer comprised of alternating units of acetylglucosamine and glucuronic acid and having a molecular weight of several million. It is found in the vitreous humor, synovial fluid, pathologic joints, group A and C hemolytic streptococci, and the skin, in which it is found in the greatest abundance. Suitable salts of hyaluronic acid that may be used in the composition of the invention include water soluble salts, preferrably the sodium salts.

Hyaluronic acid containing polymers may also be used in the composition of the invention. As used herein, "hyaluronic acid containing polymers" includes polymers made from (a) hyaluronic acid or salts thereof, and (b) a second polymer other than hyaluronic acid. The second polymer preferably is a cationic polymer, most preferably polymeric quaternary ammonium salts. Suitable hyaluronic acid containing polymers for use in the composition of the invention and methods for making such polymers are described in U.S. Patent 4,629,623. A particularly preferred hyaluronic acid containing polymer for use in the present invention is BioCare Polymer HA-24, which is a complex formed between sodium hyaluronate and Quatrisoft Polymer LM-200, a polymeric quaternary ammonium salt of hydroxyethylcellulose reacted with a lauryl dimethyl ammonium substituted epoxide. BioCare Polymer HA-24 and Quatrisoft Polymer LM-200 may be obtained from Amerchol Corporation of Edison, New Jersey.

The moisturizing and soothing agents that desirably are included in the composition of the invention

2

may be obtained from a number of commercially available sources. In particular, allantoin is commercially available from Sutton Laboratories, Inc. in Chatham, New Jersey, aloe vera gel is available from Bio-botanica, Inc. in Hauppauge, New York, and moisturizing botanical extracts are available as Phytelene Plant Extracts from International Sourcing, Inc. in Paramus, New Jersey.

As is known to persons skilled in the art, allantoin is derived from the oxidation of uric acid and hastens epithelial formation. Aloe vera gel comprises a mixture of polysaccharides, essential vitamins and minerals that are derived from the plant Aloe Barbondensis Miller. Botanical extracts useful in the invention may be derived from a variety of plants by cold extraction processes, yielding a hydroglycolic extract. A particularly preferred plant extract is Phytelene EGX-244, which is a mixture of Calendula Extract, Chamomile Extract, Linden Extract, Cornflower Extract, Matricaria Extract and Hypericum Extract.

When allantoin is present, it most preferably comprises between 0.01 to 1.0 percent by weight of shave gel composition. Aloe vera gel, when used, most preferably comprises between 0.1 to 5.0 percent by weight, and the botanical plant extracts, when used, typically comprises between 0.1 to 5.0 percent by weight of the composition.

The water used in the composition may be tap water, distilled water, or deionized water. Relatively small amounts of additional substances, including polar materials, such as lower molecular weight alcohols, methanol, ethanol, propanol, isopropanol, and the like, may also be included in the water, provided that sufficient water is present to maintain the desired physical characteristics of the gel and that the additional materials are compatible with the other materials present in the gel.

Generally, the amount of water employed in the gel may be varied depending upon the properties desired in the final product, and the nature of soap, gelling agent and other constituents of the composition. The upper limit of water that may be used is fixed as the amount that will permit the production of a satisfactory gel and a satisfactory lather at the temperatures likely to be encountered during use, while the lower limit of water that desirably is used is fixed primarily by economic considerations.

Soaps that may be used in the present invention are well known in the art and may be prepared in any conventional manner. For example, suitable soaps may be prepared by reacting a basic material such as triethanolamine directly with a higher fatty acid such as stearic, palmitic, myristic, oleic, coconut oil fatty acids, soya oil fatty acids, and mixtures of such acids. Animal fats, such as tallow, or vegetable fats (e.g., palm oil), which are rich in fatty acids may be used as the source of the acid.

The precise soap used, although not critical, has an effect on the type of gel and lather produced. Preferred soaps include the stearate and palmitate soaps, such as the potassium, ammonium, and soluble amine soaps of commercial stearic acid, it being understood that commercial stearic acid is in fact a mixture consisting primarily of stearic and palmitic acids. The triethanolamine and morpholine soaps of commercial stearic acid are particularly preferred.

Mixtures of different soaps may also be used, including mixtures that contain small amounts (preferably less than 5 percent by weight) of a soap having relatively low water solubility such as a sodium stearate soap. Soaps derived from vegetable oil may be used alone, but preferably are used in admixture with soaps derived from animal fat and/or vegetable fat. Preferably at least about 30 percent by weight, and most preferably from 60 to 100 percent of the soap, is water soluble stearate soap.

The amount of soap that is desirably used in the composition of the invention is a function generally of the particular soap used. The lower limit is the minimum amount that gives a satisfactory lather and gel, with the upper limit being fixed either by economic considerations or by the amount which will form a suitable gel at the highest temper ature likely to be encountered in use. Usually, the composition will contain from about 5 to about 35 percent of soap, and most preferably containing about 15 to 25 percent by weight of soap.

A wide variety of gelling agents, which are known in the art, may be used in the composition of the invention. Preferred gelling agents include water-soluble derivatives of naturally occurring celluloses, sucroses, and glucoses. Particularly preferred are:

(a) hydroxyethylcelluloses;
(b) hydroxypropylcelluloses;
(c) copolymers of acrylic acid and a polyallyl sucrose; and
(d) reaction products of cellulose or glucose with acids or alkylene oxides.

Such gelling agents may desirably be employed in concentrations from as low as about 0.01 percent by weight to as high as about 5.0 percent by weight; however, economics generally mitigate against higher concentrations. Preferably, the gelling aid is used in amounts of from about 0.05 to about 1.5 percent by weight of the total composition.

The post-foaming agents of the invention have a vapor pressure and are present in amounts such that, in use:

(a) when the gel is dispensed from the container, at room temperature and without rubbing or other agitation, the gel does not foam for at least a discrete period of time (e.g., for at least a second or two, preferably longer); and

(b) when the gel is rubbed between the fingers or onto the skin, a lather is produced by the volatilization of the agent.

Preferred post-foaming agents include (a) the aliphatic hydrocarbons having from 4 to 6 carbon atoms, i.e., butanes (preferably isobutane), pentanes, and hexanes, and (b) partially or wholly halogenated hydrocarbons, such as trichlorotrifluoroethane (Freon 13), and 1,2 dichloro, 1,1,2,2-tetrafluoroethane (Freon 114). Mixtures of such post-foaming agents are useful for providing the particular vapor pressure desired.

It is desirable to avoid the use of post-foaming agents that result in a burning or other undesirable sensation when the gel is applied to the skin. And, like the other components of the composition of the invention, the post-foaming agent should not be toxic.

Not only the vapor pressure of the post-foaming agent, but also the influence of the other substances present in the gel, should be considered in preparing the compositions of the invention. Typically, aliphatic hydrocarbon post-foaming agents will be used in an amount comprising at least about 0.5 percent, preferably from about 1 to about 4 percent by weight of the gel. Halogenated hydrocarbon post-foaming agents typically will be used in an amount comprising at least about 1 percent, preferably from about 2 to about 8 percent by weight of the gel. Mixtures of post-foaming agents may be used to tailor the post-foaming and other characteristics of the final product.

In addition to the components described above, the composition of the invention may also include a number of additional additives that conventionally are included in shave and/or cosmetic compositions. Such additional additives include perfumes, flavors, skin conditioners, coloring agents, preservatives, emollients, and humectants.

Techniques of the type commonly used in the cosmetics industry may be used to combine the ingredients of the shave gel composition of the present invention. Suitable techniques are described in the Example below, which is presented for the sole purpose of illustrating the invention. Unless otherwise specified, all parts and percentages are by weight.

## EXAMPLE

A composition having the following components, in the amounts specified, was prepared:

| | | Ingredients | % By Weight |
|---|---|---|---|
| | | Non-Pressurized Intermediate | |
| Phase 1 | | Deionized Water | 72.54 |
| | | Allantoin | 0.10 |
| | | Sorbitol (70% Solution) | 4.00 |
| | | Methylparaben | 0.20 |
| | | Propylparaben | 0.10 |
| Phase 2 | | Hydroxyethylcellulose | 1.00 |
| Phase 3 | | Stearic Acid | 10.00 |
| Phase 4 | | Triethanolamine 99% | 5.00 |
| Phase 5 | | Glyceryl Isostearate | 3.00 |
| Phase 6 | | Biocare Polymer HA-24 | 1.00 |
| | | Aloe Vera Gel | 1.00 |
| | | Botanical Extract Mixture: Calendula, Chamomile, Linden, Cornflower, Matricaria, and Hypericum | 2.00 |
| Phase 7 | | FD&C Blue No. 1 (1% Aqueous) | 0.06 |
| | | Fill Formula | % By Weight |
| | | Non-Pressurized Intermediate | 96.0 |
| | | n-Pentane | 4.0 |

The above composition may be made by combining the listed components in the manner described below:

The Non-Pressurized Intermediate was prepared as follows:

(a) Combine Phase 1 materials and heat to 75° C with propeller mixing to dissolve all solids.

(b) Add Phase 2 Hydroxyethylcellulose to Phase 1 and mix until a clear dispersion is formed.

(c) In a second vessel, heat Phase 3 Stearic Acid to 75° C and mix until it is all melted.

(d) Add Phase 3 material to combined Phases 1 and 2. Mix well to keep all materials uniform.

(e) Add Phase 4 Triethanolamine to combined Phases 1 through 3. Mix well at 75° C until completely saponified and the product is smooth.

(f) Cool the product to 50° C and add Phase 5 Glyceryl Isostearate.

(g) Continue to cool the product to 30° C and add the Phase 6 ingredients, Biocare Polymer HA-24, Botanical Extract Mixture and Aloe Vera Gel.

(h) Add Phase 7 color solution.

The Non-Pressurized Intermediate and the Post-Foaming Agent (n-Pentane) are combined as follows:

The Non-Pressurized Intermediate and the Post-Foaming Agent are kept in separate vessels. The Post-Foaming Agent is stored in an aerosol safe vessel under pressure. Both components are pumped separately into a "T" type junction where they are combined in the appropriate ratio. The combined components are then forced through a static mixer to avoid air entrapment and filled into appropriate containers.

**Claims**

1. A post-foaming shave gel composition comprising water, a soap, a gelling agent, a post-foaming agent, and hyaluronic acid, a hyaluronic acid containing polymer, a salt of hyaluronic acid, a salt of a hyaluronic acid containing polymer or a mixture thereof as a hyaluronic acid containing component.

2. A post-foaming shave gel comprising about 40-90% by weight water, about 4-35% by weight soap, about 0.1-5% by weight gelling agent, about 0.5-12% by weight post-foaming agent, and about 0.01-5.0% by weight of hyaluronic acid, a hyaluronic acid containing polymer, a salt of hyaluronic acid, a salt of a hyaluronic acid containing polymer or a mixture thereof as a hyaluronic acid containing component.

3. The composition of claim 1 or 2 wherein the hyaluronic acid containing component is a polymer made by reating sodium hyaluronate with a polymeric quartenary ammonium salt of hydroxyethylcellulose reacted with a lauryl dimethyl ammonium substituted epoxide.

4. The composition of any one of claims 1 to 3 further comprising allantoin, aloe vera, a botanical plant extract or a mixture thereof as a moisturizing agent.

5. The composition of claim 4 wherein the moisturizing agent comprises a botanical plant extract comprising a mixture of extracts of Calendula, Chamomile, Linden, Cornflower, Maricaria, and Hypericum.

6. A method of shaving comprising applying to the beard an effective amount of the composition of any one claims 1 to 5 under conditions in which the composition foams and, thereafter, shaving the beard.

European Patent Office

**EUROPEAN SEARCH REPORT**

Application Number

EP  89 10 7631

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | GB-A-2 160 097  (BIOMATRIX INC.)<br>* Claims 1-9; example 8 * | 1-6 | A 61 K    7/15 |
| Y | FR-A-2 094 109  (I.I. LUBOWE)<br>* Claims; example IX * | 1-6 | |
| Y | GB-A-2 188 060  (L'OREAL)<br>* Claims; page 10, lines 10-17 * | 1-6 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 18-07-1989 | WILLEKENS G.E.J. |